(19) Europäisches Patentamt
European Patent Office
Office européen des brevets

(11) Veröffentlichungsnummer: **0 173 262**
**A2**

(12) # EUROPÄISCHE PATENTANMELDUNG

(21) Anmeldenummer: **85110606.2**

(22) Anmeldetag: **23.08.85**

(51) Int. Cl.⁴: **A 61 K 31/12**, A 61 K 31/35

(30) Priorität: **24.08.84 DE 3431236**

(43) Veröffentlichungstag der Anmeldung: **05.03.86 Patentblatt 86/10**

(84) Benannte Vertragsstaaten: **AT BE CH DE FR GB IT LI LU NL SE**

(71) Anmelder: **Carnivora-Deutschland GmbH, D-7109 Jagsthausen (DE)**

(72) Erfinder: **Wagner, Hildebert, Dr., Nelkenweg 9, D-8211 Breitbrunn am Chiemsee (DE)**

(74) Vertreter: **Andrae, Steffen, Dr. et al, Patentanwälte Andrae, Flach, Haug, Kneissl Steinstrasse 44, D-8000 München 80 (DE)**

(54) **Verwendung von 1,4-Naphthochinon-Derivaten in niedrigen Konzentrationen zur Immunstimulation.**

(57) Bestimmte 1,4-Naphthochinon-Derivate der allgemeinen Formel

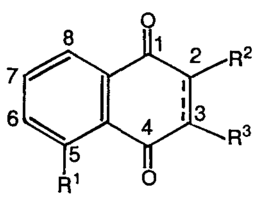

insbesondere die Verbindungen Plumbagin sowie Lapachole, erwiesen sich in hohen Verdünnungen phagocytosesteigernd und werden erfindungsgemäß in Konzentrationen im Bereich von $10^{-3}$ bis $10^{-6}$ mg/ml zur Immunstimulation verwendet.

# Verwendung von 1,4-Naphthochinon-Derivaten in niedrigen Konzentrationen zur Immunstimulation

Die Erfindung betrifft die Verwendung von 1,4-Naphthochinon-Derivaten in hochverdünnter Form zur Immunstimulation. Insbesondere betrifft die Erfindung die Verwendung von 1,4-Naphthochinon-Derivaten der allgemeinen Formel

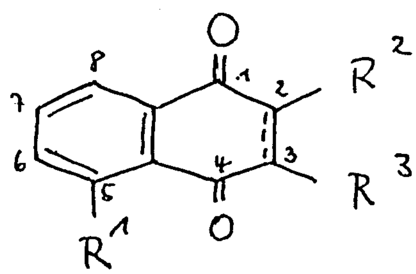

in der
$R^1$ = H, OH oder $OR^4$, wobei $R^4$ ein Alkylrest mit 1 bis 5 Kohlenstoffatomen ;
$R^2$ = ein Alkylrest mit 1 bis 6 Kohlenstoffatomen, OH oder $OR^5$, wobei $R^5$ ein Alkylrest mit 1 bis 5 Kohlenstoffatomen ist oder zusammen mit $R^3$ und den Kohlenstoffatomen in den Stellungen 2 und 3 des Naphthochinon-Gerüsts einen einfach oder doppelt alkylsubstituierten Dihydropyran-Ring bildet;
$R^3$ = H, ein Alkylrest mit 1 bis 6 Kohlenstoffatomen, ein Alkenylrest mit 2 bis 6 Kohlenstoffatomen oder bildet zusammen mit $R^5$ und den Kohlenstoffatomen in den Stellungen 2 und 3 des Naphthochinon-Gerüsts einen einfach oder doppelt alkylsubstituierten Dihydropyran-Ring; wobei
die Kohlenstoffatome in den Stellungen 2 und 3 des Naphthochinon-Gerüsts durch eine Doppel- oder Einfach-Bindung miteinander verbunden sind, mit der Maßgabe, daß eine Einfachbindung nur dann vorliegen kann, wenn die Reste $R^5$ und $R^3$ gemeinsam einen alkylsubstituierten Dihydropy-

ran-Ring bilden; in Konzentration im Bereich von $10^{-3}$ bis $10^{-6}$, bevorzugt von $10^{-3}$ bis $10^{-6}$ mg/ml, zur Immunstimulation.

Zu den angegebenen 1,4-Naphthochinonen der obigen allgemeinen Formel gehören zahlreiche natürlich vorkommende 1,4-Naphthochinon-Derivate. Derartige natürliche 1,4-Naphthochinon-Derivate werden im Rahmen der vorliegenden Erfindung bevorzugt verwendet. Besonders wichtige Vertreter sind das Plumbagin ($R^1$ = OH, $R^2$ = $CH_3$ und $R^3$ = H), das in den Wurzeln von Plumbago-Arten (Plumbago europaea = Bleiwurz) isoliert wurde und auch im rundblättrigen Sonnentau (Drosera rotundifolia) nachgewiesen wurde. Weitere besonders wichtige natürliche Vertreter der genannten 1,4-Naphthochinon-Derivate sind die in nordamerikanischen Tabebuia-Arten vorkommenden Lapachole, bei denen in der obigen allgemeinen Formel $R^1$ = H, $R^2$ = OH und $R^3$ = ein 3-Methyl-2-butenyl-Rest ($C_5$-Isopren-Seitenkette). Bei einer ringgeschlossenen Form der genannten Verbindung sind die Reste $R^2$ und $R^3$ miteinander verbunden, indem ein di-methylsubstituierter Dihydropyran-Ring gebildet wird. Die beiden Lapachole weisen somit die beiden folgenden Formeln auf:

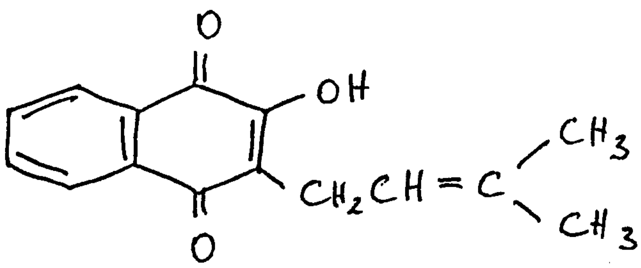
;
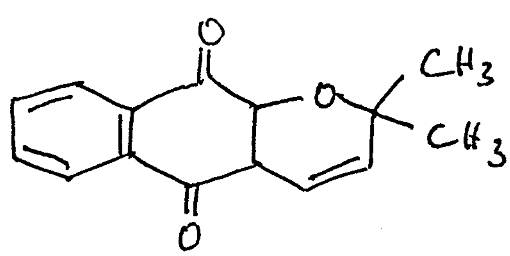

Die Erfindung betrifft die Verwendung derartiger 1,4-Naphthochinon-Derivate in den angegebenen hochverdünnten Konzentrationen zur Immunstimulation. Dabei können diese 1,4-Naphthochinon-Derivate in Form der reinen, verdünnten Chemikalie eingesetzt werden, wie auch in der Form verdünnter pflanzlicher Extrakte, die diese Verbindungen enthalten.

Die Erfindung besteht darin, 1,4-Naphthochinon-Derivate der angegebenen Form in der angegebenen Verdünnung zur Immunstimulation zu verwenden. Immunstimulation ist ein für den Fachmann mit einem präzisen Bedeutungsinhalt gefüllter Begriff, der eine Anregung des menschlichen Immunapparats unter Steigerung der unspezifischen Immunabwehr betrifft. Zum Begriff der Immunstimulation kann auf eine Reihe von Veröffentlichungen hingewiesen werden, von denen beispielsweise zu nennen sind:

Drews, J. "Möglichkeiten der Immunstimulation", Sandorama 1980, S. 12-15;

Drews, J. "A Role for Immune Stimulation in the Treatment of Microbial Infections?", Infection, 8. Jahrgang, 1980, Nr. 1, S. 2-4;

Mayr, A. et al, "Paramunität, Paramunisierung, Paramunitätsinducer", Fortschritte der Medizin, 97. Jahrgang, Nr. 27, 1979, S. 1205-1210;

Hänsel, R., "Anti-infektiöse Prophylaxe durch Immunstimulantien - unter besonderer Berücksichtigung der Phytopharmaka", Zeitschrift für angewandte Phytotherapie V/1981, S. 172-180.

Immunstimulation wird dabei im Einklang mit den genannten Veröffentlichungen im Rahmen der vorliegenden Anmeldung insbesondere als Erhöhung der Fähigkeit eines Organismus zur unspezifischen Infektabwehr verstanden, die sich insbesondere als Steigerung der Phagocytose oder Makrophagen-Aktivität zu erkennen gibt. Diese Steigerung der Phagocytose kann außerdem mit einer Anregung der Interferon-Produktion und einer Lymphozyten-Stimulierung verbunden sein, ohne daß diese Effekte für eine Immunstimulation im Sinne der vorliegenden Anmeldung tatsächlich alle gleichzeitig auftreten müssen.

Die Erfindung kann somit auch als Verwendung von 1,4-Naphthochinon-Derivaten, insbesondere der angegebenen allgemeinen Formel sowie der konkret genannten Verbindungen, in hochverdünnter Form zur künstlichen unspezifischen Steigerung der Infektabwehr bezeichnet werden.

Es sind bereits zahlreiche Substanzen bekannt, die im obigen Sinne immunstimulierend wirken. Dazu gehören neben bestimmten Zubereitungen aus Viren oder Bakterien auch zahlreiche hochmolekulargewichtige Verbindungen wie isotaktische Polyacrylsäuren, Glukane, Muramyl-peptide oder die auch im Pflanzenreich gefundenen Ubichinone. Es war bisher nicht bekannt, daß auch 1,4-Naphthochinone der obigen Typen immunstimulisierend wirken. Ein sehr wesentlicher Grund dafür ist, daß diese Immunstimulation nur bei der Anwendung der obigen Verbindungen in sehr hoher Verdünnung erhalten wird. Erhöht man die Konzentration der genannten Verbindungen, kommt es zu einem völligen Umschlagen der Wirkung, und die Verbindungen erweisen sich als zytotoxisch und zeigen je nach Verbindungstyp eine Reihe anderer physiologischer Wirkungen, die insgesamt eher als toxisch zu bezeichnen sind. In diesem höheren Konzentrationsbereich wird keine Immunstimulation erhalten, sondern sogar eine Immunsupression. Soweit Verbindungen der obengenannten Art in pflanzlichen Extrakten enthalten sind, die bereits für Heilzwecke verwendet werden, war die immunstimulierende Wirkung nicht bekannt. Sonnentauextrakte z.B., die vermutlich auch Plumbagin enthalten, wurden wegen ihrer schleimlösenden Wirkung insbesondere zur Hustenbekämpfung verwendet. Diese bekannte Verwendung eines Sonnentauextrakts , selbst wenn sie mit einer Immunstimulation verbunden sein sollte, wird ausdrücklich von der vorliegenden Erfindung nicht umfaßt.

Die vorliegende Erfindung beruht auf dem erstmaligen Nachweis einer Immunstimulation durch 1,4-Naphthochinon-Derivate der angegebenen Art. Die Immunstimulation läßt sich experimentell in vitro und in vivo klar nachweisen. Als geeignete immunologische Testverfahren dienen der Carbon Clearance-Test (CCT)-RES-Funktion (Biozzi, G. Benacerraf, B. und Halpern, B.N., Br. J. Exp.Pathol. 34, 441 (1953)); der Granulocyten-Test gemäß Brandt, L., Scand.J.Haematol. Suppl. 2 (1967), der vorzugsweise in modifizierter Form angewandt wird; sowie eine Chemoluminenszenz-Messung nach Allen (Allen, R.C. in "Bioluminescence and Chemiluminescence; Basic Chemistry and Analytical Applications", Eds: De Luca und Mc Elroy, S. 63-73, Academie Press, New York, 1981).

Vertreter der eingangs bezeichneten Substanzklasse erwiesen sich in allen diesen Testen als immunstimulierend, wobei die Teste mehrfach durchgeführt wurden. In den in vitro-Versuchen wurden maximale Phagocytose-Steigerungsraten im Bereich von 20 bis 40% bei einem Konzentrationsbereich von $10^{-4}$ bis $10^{-5}$ mg/ml erhalten. Bei den Biolumineszenz-Messungen liegen die maximalen Steigerungsraten von 40 bis 60% im Konzentrationsbereich von $10^{-3}$ bis $10^{-4}$. Beide Ergebnisse korrelieren somit gut miteinander. Im in vivo-Versuch (Carbon Clearance-Test) wurden Phagocytose-Steigerungen im Bereich von bis etwa 30% erhalten (jeweils für Plumbagin).
Die beanspruchte Wirkung kann somit als experimentell gesichert angesehen werden. Die Naphthochinone erwiesen sich dabei sowohl bei der peroralen Zufuhr als auch der parenteralen Zufuhr als wirksam. Aufgrund der vorliegenden Versuchsergebnisse lassen sich für die Behandlung des Menschens für die perorale oder parenterale Verabreichung Tagesdosen im Bereich von 0,5 bis 10 mg/Mensch und Tag, vorzugsweise im Bereich von 1 bis 5 mg/Mensch und Tag, errechnen.

Bei Konzentrationen im Bereich von $10^{-2}$ bis $10^{-1}$ schlug der beobachtete Effekt um, und in den obigen Versuchen wurde eine Phagocytosehemmung erhalten.

Nachfolgend werden repräsentative Versuche beschrieben, die die Wirksamkeit von Vertretern der eingangs genannten 1,4-Naphthochinon-Derivate zeigen:

1. Carbon Clearance-Test (CCT) - Maus-RES Funktion

Prinzip des Tests:

Partikel in einer Größenordnung von 20 -25 nm (z.B. Kohlepartikel) können aus dem Organismus nur durch Phagozytose durch Makrophagen eliminiert werden. Bei intravenöser Verabreichung von Kohlepartikel werden diese durch Phagozytose der Makrophagen in Leber (Kupffer'sche Sternzellen) und Milz aus dem Blutstrom eliminiert.

Versuchstiere:

Weibliche oder männliche $F_1$ Hybridmäuse ($B_6D_2F_1$ = CS 7 Bl/6 ♀ x DAB/2 ♂) im Gewicht von 15 - 20 g.

Kollektivgröße:

8 Tiere pro Substanz (randomisiert).

Im Vergleich dazu: nur mit Lösungs- oder Suspensionsmittel vorbehandelte Tiere.

Haltung und Fütterung:

In Makrolonkäfigen, Haltung bei 20 - 22°C. Futter (Pellet; SFI Standard) und Wasser ad libitum.

Testsubstanz

Plumbagin in Form einer Reihe von verdünnten Lösungen wurde in diesem Test eingesetzt, wobei zusätzlich zur nachfolgend beschriebenen intravenösen Verabreichung auch eine perorale Verabreichung in entsprechenden Körperdosen zur Anwendung kam.

Behandlungsmodus:

Die Prüfung der RES Aktivierung der Testsubstanz erfolgt durch einmalige oder wiederholte Verabreichung als wäßrige Lösung oder bei wasserunlöslichen Substanzen als Suspension. Die Suspension wird in einer Mischung von 0,2% Tween 80, 0,2% Carboxymethylcellulose und $H_2O$ mittels Ultraschalles (Branson Sonifier) hergestellt. Die Dauer und Stärke der Beschallung richtet sich nach den bei der Prüfung der akuten Toxizität verwendeten Werten. Die für die Behandlung vorgesehene Substanzmenge wird entweder in 4 täglichen Einzeldosen oder einmal 24 Stunden vor Testbeginn i.p. oder s.c. appliziert.

Dosierung der Prüfsubstanz:

Für die Behandlung beträgt die höchste verabreichte Gesamtdosis der Prüfsubstanz im allgemeinen 30 mg/kg KGW.

Durchführung des Carbon Clearance Tests:

Tuschesuspensionen mit einem 10%igen Gehalt an Gasruß (C 11/1431a, Fa. Günther Wagner, Hannover) wird mit einer 1%igen Gelatine-NaCl-Lösung auf einen Gehalt von 16 mg Kohle/ml verdünnt. Die Suspension wird in Wasser auf 37°C vorgewärmt. Jede Maus erhält 0,2 ml/20 g KGW intravenös verabreicht. 3, 6, 9, 12 und 15 min. nach der intravenösen Verabreichung werden durch Punktion des Orbitals plexus (Pipette tips, Micro-Selectapette, Clay Adams, Parsipanny NYUSA) 25 µl Blut entnommen. Das Blut wird in 2 ml Aqua dest. hämolysiert. Die Kohlekonzentration wird photometrisch (Photometer Zeiß PMQ II) bei einer Wellenlänge von 650 nm und einer Küvettendicke von 1 cm bestimmt. Anschließend werden die Tiere getötet und Leber- und Milzgewichte bestimmt.

Statistische Auswertung:

Die Auswertung erfolgt mittels eines EDV Programmes in dem die Eliminationskinetik der Kohlepartikel von behandelten und unbehandelten Tieren verglichen wird. Verglichen werden die Regressionskoeffizienten x) und die α-Werte:

$$\alpha = \frac{\sqrt[3]{b} \cdot KG}{\text{Leber / Milzgew.}}$$

b = Regressionskoeffizient
KG = Körpergewicht

Beurteilung der Wirksamkeit:

| Regressionskoeffizient | | DSK |
|---|---|---|
| behandelte Gruppe = unbehandelte Gruppe | | 0 |
| $\frac{\text{behandelte Gruppe}}{\text{unbehandelte Gruppe}}$ | < 1,5 | 1 |
| $\frac{\text{behandelte Gruppe}}{\text{unbehandelte Gruppe}}$ | > 1,5 | 2 |

x Der Regressionskoeffizient gibt an, um wieviel Einheiten sich y ändert wenn x um 1 Einheit zunimmt.

Bei diesen Versuchen wurden mit Plumbagin-Lösungen Phagocytosesteigerungen im Bereich von 20 bis über 30 % erhalten, wenn Lösungen der eingangs genannten Verdünnung eingesetzt wurden.

## 2. Granulozyten-Test (mod. nach Brandt)

### Methodik

In 20 ml Spritze 1 Tropfen Heparin (Thrombophob) und ca. 10 ml Blut abnehmen und mit 1,5% Dextran T 500 im Verhältnis 2:1 mischen. Kanüle auf der Spritz umbiegen und 30 Minuten bei 25°C absetzen lassen (ca. 2-3 ml Blut für Eigenserum abnehmen).

Den Überstand (Leukozytensuspension) in steriles Röhrchen überspritzen; 7 Minuten bei 500 U. zentrifugieren.

Überstand weggießen, Bodensatz (Leukozyten) 2 mal mit RPM I-Puffer bei 500 U. waschen.

### Zellzählung

Granulozyten mit ca. 5 ml Puffer auffüllen und Zellen auf $5 \times 10 \times 10^6$ einstellen. 25 µg Zellsuspension in 0,475 ml 3%iger Essigsäure mischen. In Neubauerkammer 4 Quadrate auszählen (100 Zellen/Kammer). Summe mal 50 mal 1000 = Granulozyten/1 ml.

### Herstellung der Inkubationsmixtur

Poolserum und Hefesuspensin aus Gefrierfach auftauen.
Eigenserum 1 : 5 mit 0,9% NaCl verdünnen
Poolserum 1 : 5 mit 0,9% NaCl verdünnen
Hefesuspension unverdünnt.

### geteste Substanz

Plumbagin in Form einer Reihe kontinuierlich verdünnter Lösungen im Konzentrationesbereich von $10^{-1}$ bis $10^{-5}$mg/ml.

Mischen

| | |
|---|---|
| 0,2 ml Zellsuspension | 0,2 ml Zellsuspension |
| 0,2 ml Hefesuspension | 0,2 ml Hefesuspension |
| 0,2 ml Eigenserum | 0,2 ml Poolserum |

Beide Röhrchen im Brutschrank bei 37°C 10 Minten rotieren lassen. Je 500 µl auf die beschrifteten Objektträger auftragen und in feuchter Kammer weitere 20 Minuten bzw. 50 Minuten inkubieren lassen. Überstand vorsichtig mit NaCl abspülen. Föhntrocknen. Färben.

Färben (nach Pappenheim)
5 Min. May-Grünwald
2 Min. Aqua dest.
15 Min. Giemsa-Lsg. ( 5 ml/180 ml Aqua dest.)

Lufttrocknen lassen

Auswertung des Tests mit Mikroskop 1200 x Vergrößerung
Es werden 100 Granulocyten ausgezählt und bestimmt, wie viele Hefepartikel ein einzelner Granulocyt (im Durchschnitt) phagozytiert. Daraus ergibt sich der Phagozytose-Index.

$$\%\text{Phagozytose-Steigerung} = \frac{\text{Phag.Index d.Subst.} - \text{Phag.Index d.Kontrolle}}{\text{Phag.Index d. Kontrolle}} \times 100$$

Ergebnisse

Die maximalen Phagocytosesteigerungsraten im Bereich von 20 bis 40 % wurden im Konzentrationsbereich von $10^{-4}$ bis $10^{-5}$ mg/ml erhalten. Im Konzentrationsbereich von höheren Konzentrationen von $10^{-1}$ bis $10^{-2}$ mg/ml wurde eine ausgeprägte Phagocytosehemmung beobachtet.

3. Chemolumineszenz-Messung (mod. nach ALLEN )

Herstellung der Lösungen:

a) PBS-Puffer ohne $Ca^{2+}$ und $Mg^{2+}$ (steril) (Fa. Biochrom, Berlin)

b) Veronal-gepufferte Kochsalzlösung mit $Ca^{2+}$, $Mg^{2+}$, Glucose und Albumin
Stammlösung: 8.3 g NaCl, 1.02 g Natrium, 5,5-Diäthylbarbiturat (Veronal), 102 mg $MgCl_2$, 22 $mgCaCl_2$ in 150 ml $H_2O$ (bidest.) lösen, + 3.5 ml 1M HCL auf 200 ml mit $H_2O$ (bidest.) auffüllen.
Von dieser Stammlösung werden 40 ml mit 140 ml $H_2O$ (bidest.) verdünnt, mit 1M HCL auf pH 7.0 eingestellt und mit $H_2O$ (bidest.) auf 200 ml aufgefüllt. Anschließend werden 200 mg Rinder-Serumalbumin (rein ! Fa. Boehringer) und 200 ml Glucose zugefügt und das Ganze sterilfiltriert.

c) Lucigenin:
5mM-Lösung: 25.5 mg/10 ml $H_2O$ (bidest.)
1mM-Lösung: 5mM-Lösung mit $H_2O$ (bidest.) im Verhältnis 1:5 verdünnt

d) Opsoniertes Zymosan (Fa. Berthold, München)
25 mg Zymosan/10 ml 0.9% NaCl, 30 min bei 90°C im Wasserbad kochen. Nach dem Abkühlen wird in 2 ml Portionen verteilt und eingefroren
2 ml Zymosan und 2 ml Serum 30 min werden bei Raumtemperatur inkubiert, zentrifugiert, mit physiologischer Kochsalzlösung gewaschen und danach mit je 2 ml 0.9 ml 0.9% NaCl versetzt.

Als Testlösungen dienten Lösungen in einer kontinuierlichen Verdünnungsreihe im Konzentrationsbereich von $10^{-1}$ bis $10^{-6}$ mg/ml Plumbagin/Wasser.

Gewinnung von polymorphkernigen Leucocyten (PMNL):

20 ml Nativblut wird mit 30 mg EDTA als Gerinnungshemmer versetzt .

Erythrocyten-Sedimentation mit Dextran:

15 ml Blut und 7 ml Dextran werden in einer Spritze gemischt und 30 min. bei Raumtemperatur belassen (Kanüle umbiegen). Der leukozytenhaltige Plasma-Überstand wrd langsam in ein steriles Zentrifugenröhrchen überführt und 10 min bei 1000 Upm in der Laborzentrifuge zentrifugiert. Der Niederschlag besteht aus Leucozyten und Restanteilen von Erythrocyten.

Hypotone Lyse zur Entfernung der restlichen Erythrozyten:

Der Niederschlag wird in 20 ml 0.2% Kochsalzlösung suspendiert und 20 sec auf dem Whirlimix geschüttelt (Flüssigkeitsfilm soll an der Wand des Zentrifugenglases entlanglaufen), dann wird 20 ml 1.6% NaCl zugegeben und geschüttelt (Zellen sind jetzt wieder in physiolgischem Medium). Es wird nun 10 min bei 2000 Upm zentrifugiert und der Überstand abgesaugt. Der Niederschlag (ND) enthält noch Erythrocyten, so daß zweimaliges Wiederholen der hypotonen Lyse notwendig ist. Der ND wird mit PBS ohne $Ca^{2+}/Mg^{2+}$ und anschließend in 2 ml PBS ohne $Ca^{2+}/Mg^{2+}$ suspendiert. Die gut suspendierten Zellen werden in ein neues Röhrchen pipettiert und gezählt.

Zellzahl:

Die PMNL-Suspension 1:10 wird mit Türks-Lösung verdünnt und die vorhandenen Zellen ausgezählt. Die Suspension wird mit PBS ohne $Ca^{2+}/Mg^{2+}$ auf 1000 Zellen/$\mu$l verdünnt.

Chemolumineszenz-Messung:

- 630 $\mu$l Veronal-gepuffertes NaCl
- 30 $\mu$l PMNL-Suspension
- 200 $\mu$l PBS ohne $Ca^{2+}/Mg^{2+}$ bzw. in PBS gelöste Probe
- 100 $\mu$l Lucigenin 1mM)

Man läßt 10 min im 6-Kanal-Biolumat (Fa.Beckman) bei 37°C temperieren, gibt 40 µl opsoniertes Zymosan zu und startet die Messung. Nach 60 min wird gestoppt.

Ergebnisse

Maximale Steigerungsraten wurden im Plumbagin-Konzentrationsbereich von $10^{-3}$ bis $10^{-4}$ mg/ml erhalten, wobei die Steigerungen 40 bis 60 % betrugen. Diese Ergebnisse korrelieren gut mit den Ergebnissen des Tests gemäß 2).

Im Konzentrationsbereich von höheren Konzentrationen von $10^{-1}$ bis $10^{-2}$ mg/ml wurde eine Phagocytosehemmung sowie eine Immunsuppression bzw. Cytotoxicität erhalten.

0173262

Dipl.-Chem. Dr. Steffen ANDRAE
Dipl.-Phys. Dieter FLACH
Dipl.-Ing. Dietmar HAUG
Dipl.-Chem. Dr. Richard KNEISSL
PATENTANWÄLTE
Steinstr. 44, D-8000 München 80

23. AUG. 1985

Anmelder: Carnivora-Deutschland GmbH
7109 Jagsthausen

Az:247 /AS/sc

---

Verwendung von 1,4-Naphthochinon-Derivaten in niedrigen Konzentrationen zur Immunstimulation

---

## Patentansprüche

1 1. Verwendung von 1,4-Naphtochinon-Derivaten in hochverdünnter Form zur Immunstimulation.

2. Verwendung nach Anspruch 1, wobei 1,4-Napthochinon-Derivate der allgemeinen Formel

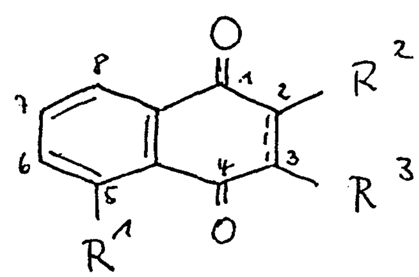

in der
$R^1$ = H, OH oder $OR^4$, wobei $R^4$ ein Alkylrest mit 1 bis 5 Kohlenstoffatomen ;

0173262

$R^2$ = ein Alkylrest mit 1 bis 6 Kohlenstoffatomen, OH oder $OR^5$, wobei $R^5$ ein Alkylrest mit 1 bis 5 Kohlenstoffatomen ist oder zusammen mit $R^3$ und den Kohlenstoffatomen in den Stellungen 2 und 3 des Naphthochinon-Gerüsts einen einfach oder doppelt alkylsubstituierten Dihydropyran-Ring bildet; $R^3$ = H ein Alkylrest mit 1 bis 6 Kohlenstoffatomen, ein Alkylenrest mit 2 bis 6 Kohlenstoffatomen oder bildet zusammen mit $R^5$ und den Kohlenstoffatomen in den Stellungen 2 und 3 des Naphthochinon-Gerüsts einen einfach oder doppelt alkylsubstituierten Dihydropyran-Ring; wobei die Kohlenstoffatome in den Stellungen 2 und 3 des Naphthochinon-Gerüsts durch eine Doppel- oder Einfach-Bindung miteinander verbunden sind, mit der Maßgabe, daß eine Einfachbindung nur dann vorliegen kann, wenn die Reste $R^5$ und $R^3$ gemeinsam einen alkylsubstituierten Dihydropyran-Ring bilden;
verwendet wird.

3. Verwendung eines 1,4-Naphthochinon-Derivats nach Anspruch 2, wobei in der allgemeinen Formel $R^1$ = OH oder $OR^4$; $R^2$ = ein Alkylrest mit 1 bis 6 Kohlenstoffatomen und $R^3$ = H.

4. Verwendung von 1,4-Naphthochinon-Derivaten nach Anspruch 2, wobei in der allgemeinen Formel $R^1$ = OH, $R^2$ = $CH_3$ und $R^3$ = H.

5. Verwendung von 1,4-Naphthochinon-Derivaten nach Anspruch 2, wobei in der allgemeinen Formel $R^1$ = H; $R^2$ = OH oder $OR^5$, wobei $R^5$ ein Alkylrest mit 1 bis 5 Kohlenstoffatomen ist oder zusammen mit $R^3$ einen Ring der folgenden Formel bildet

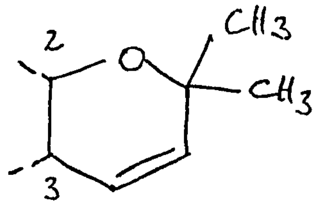 ;

und

$R^3$ : = ein Alkylrest mit 1 bis 6 Kohlenstoffatomen, ein 3-Methyl-2-butenyl-Rest oder bildet zusammen mit $R^5$ einen Ring der Formel

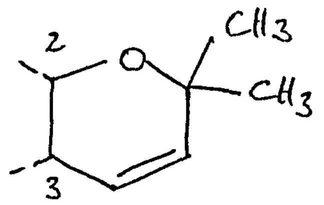

.

6. Verwendung von 1,4-Naphthochinon-Derivaten nach einem der Ansprüche 1 bis 5 im Konzentrationsbereich von $10^{-3}$ bis $10^{-6}$ mg/ml.